(19) Europäisches Patentamt
European Patent Office
Office européen des brevets

(11) EP 1 476 196 B1

(12) EUROPEAN PATENT SPECIFICATION

(45) Date of publication and mention of the grant of the patent:
**22.11.2006 Bulletin 2006/47**

(21) Application number: **03706620.6**

(22) Date of filing: **11.02.2003**

(51) Int Cl.:
*A61K 47/26* (2006.01) *A61K 47/44* (2006.01)
*A61K 47/34* (2006.01) *A61K 47/32* (2006.01)
*A61K 31/55* (2006.01)

(86) International application number:
**PCT/EP2003/050014**

(87) International publication number:
**WO 2003/068266 (21.08.2003 Gazette 2003/34)**

(54) **ORAL SOLID SOLUTION FORMULATION OF A POORLY WATER-SOLUBLE ACTIVE SUBSTANCE**

FESTE LÖSUNG EINER SCHWERWASSERLÖSLICHEN SUBSTANZ ZUR ORALEN VERABREICHUNG

SOLUTION SOLIDE ORALE D'UNE SUBSTANCE ACTIVE FAIBLEMENT HYDROSOLUBLE

(84) Designated Contracting States:
**AT BE BG CH CY CZ DE DK EE ES FI FR GB GR HU IE IT LI LU MC NL PT SE SI SK TR**
Designated Extension States:
**LT LV RO**

(30) Priority: **14.02.2002 EP 02075623**

(43) Date of publication of application:
**17.11.2004 Bulletin 2004/47**

(73) Proprietor: **Solvay Pharmaceuticals B.V.**
**1381 CP Weesp (NL)**

(72) Inventor: **GORISSEN, Henricus, R.M.**
**NL-1381 CP Weesp (NL)**

(74) Representative: **Verhage, Marinus**
**Octrooibureau Zoan B.V.**
**P.O. Box 140**
**1380 AC Weesp (NL)**

(56) References cited:
**EP-A- 0 292 050**
**EP-A- 0 733 642**
**EP-A- 0 830 863**
**WO-A-00/00179**
**WO-A-00/48601**
**WO-A-01/03699**
**WO-A-98/33512**
**US-A- 4 325 970**
**US-A- 5 281 420**

EP 1 476 196 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

The present invention relates to an oral solid solution formulation for a poorly water-soluble active substance of structure (I). More in particular the invention relates to a solid solution formulation of a poorly soluble active substance for which the bio-availability is strongly enhanced.

Solid solution formulations, which normally are in the form of gelatine capsules, are known in the art. EP 0001822 describes pharmaceutical formulations in the form of hard gelatine capsules filled with a liquid excipient which contains the active substance and which solidifies into a solid composition or into a thixotropic gel. US 4,795,643 discloses a solid solution formulation with a delayed release of the active substance. The delayed release is caused by the use of special polymers such as acrylate polymers or etherified celluloses.

Various active substances have a very poor solubility in water. When these active substances are administered to the body, they often have a poor bio-availability due to the poor solubility in the digestive fluid. In order to solve this problem several methods were developed, such as micronization, inclusion in cyclodextrines, the use of inert water-soluble carriers, the use of solid dispersions (WO 00/00179), or nanocrystalline or amorphous forms of an active substance.

The effect of the above mentioned methods on the bio-availability often depends on the properties of the active substance. Further the dosage forms developed until now have often drawbacks, such as poor thermodynamic stability, critical or difficult production processes or poor batch-to-batch reproducibility.

It is the objective of the present invention to provide an oral formulation for a poorly soluble active substance of structure (I) with a significant increase in bio-availability compared with said active substance in a traditionally formulated form. It is a further objective of the present invention to provide a formulation which can be prepared using normal formulation procedures and equipment, so that no large investments are necessary.

This objective can be achieved, according to the present invention, by an oral immediate release formulation with enhanced bio-availability comprising a solid homogeneous and thermodynamically stable solution of a poorly water-soluble biologically active substance of structure (I), characterised in that said solid solution comprises

a) the active substance (I) in an amount of up to 50% of the total weight of the formulation,
b) a non-ionic hydrophilic surfactant ingredient, which is in the liquid form between 15° and 30°C, in an amount of between 20% and 70 % of the total weight of the formulation and
c) a pharmaceutically acceptable organic polymer or mixture of polymers, which polymer or mixture of polymers is in a liquid form above 60°C and in a solid form below 30°C, in an amount of between 5% and 70% of the total weight of the formulation, and
d) optionally comprises a disintegrating agent in an amount of between 1% and 10% of the total weight of the formulation.

The following definitions are provided to facilitate understanding of certain terms used within the framework of the present application.

Immediate release refers to a release of at least 75 % of the drug in a dissolved form from the dosage form within 90 minutes.

Thermodynamically stable refers to the absence of significant physical or chemical changes of the product that might affect the quality of the product during storage for a period up to 5 years under ambient conditions.

With poorly water-soluble is meant that the aqueous solubility of the active substance is less than 1 in 1000. This means that according to the pharmacopoeial definitions substances that are categorised as "very slightly soluble", "practically insoluble" and "insoluble" are included in this definition (USP 24/NF 19, page 10; January 2000).

The term non-ionic hydrophilic surfactant refers to those amphiphilic substances that are soluble in water (they have higher HLB values), posses surface activity and are not ionised in aqueous solutions (H. Auterhoff, Worterbuch der Pharmazie, Wissenschafliche Verlagsgesellschaft GmbH, Stuttgart 1981, page 192).

With HLB value is meant a value on a scale from 0 to 20, that is assigned to each surfactant based on the relative proportions of the hydrophilic and hydrophobic part of the molecule. Oil soluble surfactants have low HLB values, whereas water soluble surfactants have higher HLB values.

The HLB value is calculated as:

$$HLB = 20(1-M_o/M)$$

In which M is the molecular weight of the molecule and $M_o$ is the molecular weight of the hydrophobic part of the molecule.

The ratio between the active substance in the formulation and the non-ionic hydrophilic surfactant is between

1 : 0.75 and 1 : 5, preferably between 1 : 1.5 and 1 : 4 and most preferred is 1 : 3. The ratio between the non-ionic hydrophilic surfactant and the pharmaceutically acceptable polymer or mixture of polymers is between 1 : 4 and 1 : 0.05, preferably between 1 : 1.5 and 1 : 0.1 and most preferred is approximately 1 : 0.75.

The non-ionic hydrophilic surfactant ingredient is preferably selected from the group consisting of polyoxyethylene glycol sorbitan fatty acid esters (polysorbates) and non hydrogentated polyoxyethylene castor oil derivatives, said surfactants having a hydrophilic-lipophilic balance (HLB) value of between 14 and 16. Polyoxyethylene glycol polysorbates are commercially available from ICI Inc., and are known under the trademark Tween®. For the present invention Tween® 40, Tween® 60 or Tween® 80 are preferred. The most preferred compound is Tween® 80. Non hydrogenated polyoxyethylene castor oil derivatives are commercially available from the BASF Corporation under the trademark Cremophor®. The most preferred compound for the present invention is Cremophor® EL.

In one embodiment the pharmaceutically acceptable organic polymer or mixture of polymers is consisting for a major part of polyethylene glycol (PEG) or a mixture of polyethylene glycols. PEGs are condensation polymers of ethylene oxide, commercially available from Union Carbide Corporation under the trade name Carbowax®. Preferred PEG's are those with a molecular weight of between 1000 and 50000 Daltons. More preferred are PEG's having a molecular weight between 4000 and 10000 Daltons. The most preferred PEG has a molecular weight of about 6000 Daltons.

In a further embodiment of the invention the pharmaceutically acceptable organic polymer or mixture of polymers is consisting for a major part of polyvinyl pyrrolidone (PVP) or a mixture of poyvinyl pyrrolidones, commercially available from BASF under the trademark Kollidon® having approximate molecular weights of 2500 up to 3000000 Daltons.

In an even further embodiment of the invention the pharmaceutically acceptable organic polymer or mixture of polymers is consisting for a major part of polyvinyl alcohol (PVA) or a mixture of polyvinyl alcohols, commercially available from Shin-Etsu Chemical Co under the trademark Poval® having approximate molecular weights of 30000 up to 200000 Daltons.

The formulation optionally comprises a disintegrating agent in an amount of between 1% and 10% of the total weight of the formulation. Normally a disintegration agent is not necessary, but in some cases it may be advantageous to add a small amount of such an agent in order to increase the dissolution of the formulation because of swelling and to increase the water transport into the formulation when contacting the dissolution media. An example of a disintegrating agent is Primojel®, commercially available, from Penwest Pharmaceuticals. Other disintegrating agents that can be used are Ac-di-Sol® commercially available from FMC, Kollidon CL®, commercially available from BASF or Polyplasdone XL®, commercially available from ISP.

An especially preferred dosage form for the above formulation consists of a hard gelatin capsule into which the homogeneous melt mixture is filled and allowed to solidify in situ. Another dosage form composition is made by filling the melt mixture into soft, elastic gelatin capsules or by forming molded tablets, e.g. by filling the melt mixture into tablet molds, or shaping partially solidified melt mixtures into tablet shapes, like it is performed by the melt extrusion process of Knoll AG, Ludwigshafen, BRD.

The active substance is normally used in an amount between about 0.1 and 50% by weight, preferably in an amount between 1 and 50% by weight and more preferably in an amount between about 10 and 50% by weight.

The class of active substances which are poorly soluble in water and for which the present invention is useful are the substances described in EP0733642 with the general formula

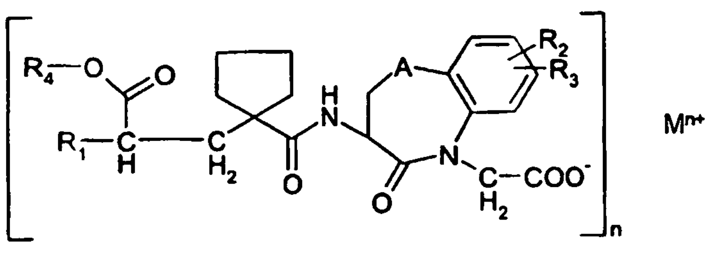

(I)

wherein:

$R_1$ is a selected from the group consisting of $(C_1-C_6)$alkoxy$(C_1-C_6)$alkyl which may be substituted by a $(C_1-C_6)$ alkoxy, phenyl-$(C_1-C_6)$-alkyl and phenyloxy-$(C_1-C_6)$-alkyl wherein the phenylgroup may be substituted with $(C_1-C_6)$

alkyl, ($C_1$-$C_6$)alkoxy or halogen, and naphtyl-($C_1$-$C_6$)-alkyl,
$R_2$ and $R_3$ are both independently hydrogen or halogen,
$R_4$ is a biolabile ester forming group,
M is a metal ion, preferably a bivalent metal ion,
n is 1, 2 or 3.
A is $CH_2$, O or S

($C_1$-$C_4$)-alkyl is defined as a straight or branched alkyl group consisting of between 1 and 4 carbon atoms. ($C_1$-$C_4$)-alkoxy is defined as a straight or branched alkoxy group consisting of between 1 and 4 carbon atoms.

Therefore the present invention is also related to a solid solution formulation as described above of a poorly water soluble compound of formula I. M is preferably a $Li^+$, $Mg^{2+}$, $Zn^{2+}$ or a $Ca^{2+}$ ion and most preferrably a $Ca^{2+}$ ion, $R_1$ is preferably phenylethyl, $R_2$ and $R_3$ are preferably hydrogen and $R_4$ is preferably ethyl. The preferred compound is the calcium salt of 1H-1-Benzazepine-1-acetic acid, 3-[[[1-[2-(ethoxycarbonyl)-4-phenylbutyl]cyclopentyl]carbonyl]amino]-2,3,4,5-tetrahydro-2-oxo-. The most preferred compound is said compound in its 3S,2'R form. This compound is referred to as Compound S-Ca; The corresponding acid (1H-1-Benzazepine-1-acetic acid, 3-[[[1-[2-(ethoxycarbonyl)-4-phenylbutyl]cyclopentyl]carbonyl]amino]-2,3,4,5-tetrahydro-2-oxo-) is referred to as Compound S-H and the corresponding S-α-methylbenzylamine salt is referred to as Compound S-Mba.

The formulation described above can be prepared using conventional formulation procedures and equipment. Therefore it is another aspect of the present invention to provide a method of preparing a formulation as described above, characterized in that a) the non-ionic hydrophilic surfactant ingredient is mixed with the pharmaceutically acceptable organic polymer or mixture of polymers at between 50-100 °C, preferably between 60 and 70°C, b) the active ingredient is added and dissolved at said temperature, c) the resulting mixture is optionally filled into a capsule and d) resulting mixture is solidified at room temperature.

Alternatively the non-ionic hydrophilic surfactant ingredient, the pharmaceutically acceptable organic polymer or mixture of polymers and the active substances are mixed together and heated to a temperature of between 50 and 100 °C, preferably between 60 and 70 °C until a clear solution is obtained, optionally followed by filling of the solution into a capsule.

The following examples are only intended to further illustrate the invention, in more detail, and therefore these example are not deemed to restrict the scope of the invention in any way.

**EXAMPLES.**

**Example 1. Effect of type and quantity of surfactant on release.**

<u>PREPARATION OF THE FORMULATIONS.</u>

The non-ionic hydrophilic surfactant, Tween 80 or Cremophor EL is heated together with a hydrohilic polymer, PEG 6000 up to a temperature above 60°C. The active substance is added and dissolved in the melt at said temperature. The resulting solution is filled into capsule size 0 (zero). The solution solidifies in the capsule at room temperature.
Increasing quantities of a surfactant together with a poorly water soluble active substance and the hydropholic polymer were composed. The compositions are given in Table 1 for liquid filled capsules which contain 50 mg active substance. The effect of the amount and the effect of type of surfactant on the release of the active substance from the liquid filled capsule was determined.

**Table 1.** Effect of quantity and type of surfactant on the dissolution of the active substance (Amounts are expressed in %)

| Materials | Composition in % | | | | | | |
|---|---|---|---|---|---|---|---|
| | A | B | C | D | E | F | G |
| Active substance*) | 16.0 | 16.0 | 16.0 | 16.0 | 16.0 | 16.0 | 16.0 |
| Tween 80® | 0.0 | 12.0 | 24.0 | 48.0 | n.a. | n.a. | n.a. |
| Cremophor EL® | 0.0 | n.a. | n.a. | n.a. | 12.0 | 24.0 | 48.0 |

(continued)

| Materials | Composition in % | | | | | | |
|---|---|---|---|---|---|---|---|
| | A | B | C | D | E | F | G |
| PEG 6000 | 84.0 | 72.0 | 60.0 | 36.0 | 72.0 | 60.0 | 36.0 |
| *) calcium salt of 1H-1-Benzazepine-1-acetic acid, 3-[[[1-[(2R)-2-(ethoxycarbonyl)-4-phenylbutyl]cyclopentyl] carbonyl]amino]-2,3,4,5-tetrahydro-2-oxo-, (3S)-. (Compound S-Ca)<br>n.a.: not applicable | | | | | | | |

IN-VITRO DISSOLUTION TESTING

Dissolution system.

Dissolution testing of the liquid filled capsules is performed in artificial gastrointestinal fluids of 37°C using USP II apparatus using a paddle speed of 100 rpm and a sinker for each capsule The dissolution is tested in a sequential range of increasing pH of the medium starting with 400 ml pH 2, prepared from 400 ml 0.01 N hydrochloric acid. One hour after starting the dissolution 15 ml of the medium is withdrawn and the pH of the buffer is changed into pH 4.5 by adding 88.5 ml 0.05 N glacial acetic acid and 211.5 ml 0.05 N sodium acetate solution. After 30 minutes 5 ml of the medium is withdrawn and the pH of the buffer is changed into pH 6.8 by adding 180 ml 0.2 N disodium hydrogen phosphate and 120 ml 0.2 N potassium dihydrogen phosphate. After 2½ hours 5 ml of the medium is withdrawn and the dissolution test is stopped.

Chromatographic system.

A high-performance liquid chromatographic system equipped with a thermostated column compartment, UV-absorbance detector with adjustable wavelength and integrating system is used. The analytical column (length 3 cm, internal diameter 3 mm) is a C18-modified silica, preferably Inertsit® ODS-3 column, particle size 3 µm. The mobile phase consists of a degassed mixture of 350 ml of water containing 800 mg of ammonium acetate and 800 µl of trifluoracetic acid and 650 ml of acetonitril. The flow rate is 0.5 ml/min. The column temperature is 40 °C. The injection volume is 5 µl and the wavelenght of the UV absorbance detector is 236 nm. For external standardisation 0.12 mg of Compound S-Mba RS is dissolved in 1 ml mobile phase. The quantity of dissolved Compound S-H, expressed in percent relative to the label claim, is given by the equation 1:

$$\%dissolved = \frac{I_{sa} \times m_{st} \times V_{sa} \times C \times 0.8152}{I_{st} \times V_{st} \times LC}$$

**Equation 1 Calculation of the quantity dissolved Compound S-H.**

in which:

| | |
|---|---|
| $I_{st}$ | = peak area of Compound S-H in the standard chromatogram; |
| $I_{sa}$ | = peak area of Compound S-H in the sample chromatogram; |
| $V_{st}$ | = dilution volume of the standard solution, in ml (= 50 ml); |
| $V_{sa}$ | = dilution volume of the sample solution, in ml (= 400, 700 and 1000 ml); |
| $m_{st}$ | = weighed quantity of Compound S-Mba RS, in mg; |
| C | = purity of Compound S-Mba RS, in % m/m; |
| LC | = label claim of the analysed capsule, expressed as Compound S-H. |
| 0.8152 | = ratio between the molecular masses of the Compound S-H and Compound S-Mba. |

The dissolution results of the formulations A, B, C and D (see Table 1), containing Tween 80 as surfactant as determined by the above mentioned HPLC method are given in Figure 1 (♦ = 0%, ■ = 12%; ▲ = 24% and • = 48% Tween 80). The dissolution results of the formulations E, F and G (see Table 1) containing Cremophor EL as surfactant are given in Figure 2 (♦ = 0%; ■ = 12%; ▲ = 24% and •= 48% Chremophor EL).

From the results given in Figure 1 and Figure 2 it is clear that the release of the active substance from liquid filled capsules is determined by the amount of hydrophilic surfactant used in the composition. The amount of released active substance increases with increasing amounts of surfactant.

More specifically it can be seen that the release of the active substance in pH 2 (release data during the first 60 minutes of the dissolution test) is determined by the amount of surfactant in the composition. The pH change from 2 into 4.5 (release data during the next 30 minutes) improves the release of the active substance from the compositions which contain the hydrophilic surfactant at a low level. At the end of the pH 5 period it is observed that the active substance is completely dissolved when the composition contains at least 12 % surfactant Cremophor EL or 24 % Tween 80. Finally the pH change from 4.5 into 6.8 does not influence the release data anymore. The active substance remains completely dissolved when using at least 12 % Cremophor EL or 24 % Tween 80.

**Example 2. Effect of type of hydrophilic polymer on release.**

The hydrophilic polymer in the liquid filled capsules can be a polyethylene glycol product. The influence of the molecular weight of this polymer on the dissolution is tested in the compositions shown in Table 2. The formulations were prepared as described in Example 1.

**Table 2.** Composition with different types of poly ethylene glycol. (n.a. = not applicable)

| Material | Composition (%) | | |
|---|---|---|---|
| | D | H | J |
| Active substance* | 16 | 16 | 16 |
| Tween 80 | 48 | 48 | 48 |
| PEG 4000 | n.a. | 36 | n.a. |
| PEG 6000 | 36 | n.a. | n.a. |
| PEG 50000 | n.a. | n.a. | 36 |
| * Compound S-Ca; n.a.= not applicable | | | |

Dissolution testing is performed was described in Example 1. The dissolution results of the composition with Tween 80 and with different types of polyethylene glycol is given in Figure 3 (♦ =PEG 4000; ■ = PEG 6000; ▲ = PEG 50000). It is clearly shown that the active substance release from the composition containing PEG 4000 and PEG 50000 is comparable but in comparison with PEG 6000 delayed, however without being sustained release formulations.
The most preferred hydrophilic polymer is PEG 6000 because PEG 4000 will cause sooner leakage from the capsules due to its lower melting point. On the other hand PEG 50000 is difficult to handle because of the relatively high viscosity of this material in the molten phase.
The influence of the type of hydrophilic polymer is also demonstrated with capsule formulations containing Cremophor EL as surfactant. The surfactant Tween 80 of the previous examples, composition D, H and J is exchanged with the same amount of Cremophor EL The dissolution test is carried out as described previously. The dissolution results of liquid filled capsules containing 48 % Cremophor EL are given in Figure 4 (♦ =PEG 4000; ■ = PEG 6000; ▲ = PEG 50000). Figure 4 clearly shows that the active substance release from liquid filled capsules containing the hydrophilic polymers PEG 4000 and PEG 50000 is comparable but delayed in comparison with PEG 6000. The formulations can, however, not be regarded to be sustained release formulations.

**Example 3. Effect of different cations on the release of the active substance.**

The most preferred $Ca^{2+}$ ion in the formula of the active substance is replaced by several other metal ions like $Mg^{2+}$, $Na^+$ and $Li^+$. These active substances are formulated according to composition D in Table 1. This means the formulations contain 16 % active substance, 48 % Tween 80 and 36 % PEG 6000. The preparation of the formulations is in accordance with the method described for Example 1. The dissolution results of the active substance with the $Ca^{2+}$, $Mg^{2+}$, $Na^+$ or $Li^+$ ion are given Figure 5. (• = $Ca^{2+}$, 0 = $Mg^{2+}$, = $Na^+$ or Δ = $Li^+$). From the dissolution results it can be seen that said cations do not affect the release of the active substance. The profiles in pH 2, pH 4.5 and pH 6.8 are comparable.

**Example 4. Bio-availability study.**

A cross-over study in 15 male subjects is performed to test the bio-availability of the liquid filled hard gelatin

capsule. Compound S-Ca (Formulation I and III) or Compound S-H (Formulation II) is used as the drug substance. The subjects are administrated with the following formulations: (I) 2 x 103.7 mg compound S-Ca (corresponding to 100 mg compound S-H) liquid filled in hard gelatin capsule prepared according to Example 1 with the composition D, (II) 2 x 100 mg compound S-H in hard gelatin capsule as a 25 % m/m powder blend on tricalcium phosphate, (III) 8 x 25 mg plain tablet, consisting of 25.94 mg Compound S-Ca (corresponding to 25 mg compound S-H), 172 mg of micro crystalline cellulose PH101, 172 mg of mannitol-25, 8 mg of hydroxy propyl methyl cellulose E5, 20 mg of sodium starch glycolate and 2 mg of sodium stearyl fumarate. From the mean plasma levels shown in Figure 6 the results as given in Table 3 are obtained.

**Table 3** Results cross-over study in 15 male subjects.

| Formulation | Cmax ratio | Relative bio-availability |
|---|---|---|
| I | 2.7 | 1.8 |
| II | 1 | 1 |
| III | 1.9 | 1.5 |

The results from Table 3 indicate that the bio-availability of the drug substance from the liquid filled hard gelatin capsule containing 103.7 mg of Compound S Ca, 311 mg Tween 80 and 234 mg polyethylene glycol 6000 improves with 80 % in comparison with the bio-availability of formulation III in which the Compound S-H is adsorbed on tricalcium phosphate as a 25 % m/m powder blend and with 20 % in comparison with the plain tablet of Compound S-Ca.

## Claims

1. An oral immediate release formulation with enhanced bio-availability comprising a solid homogeneous and thermodynamically stable solution of a poorly water-soluble biologically active substance, **characterised in that** said solid solution comprises:

   a) a compound of the general formula

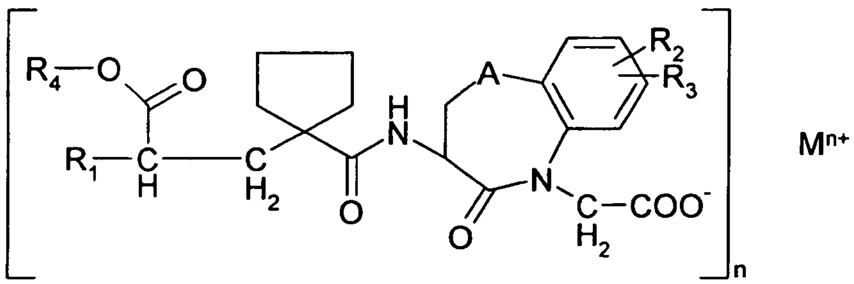

(I)

wherein:

$R_1$ is a selected from the group consisting of $(C_1-C_6)$alkoxy$(C_1-C_6)$alkyl which may be substituted by a $(C_1-C_6)$alkoxy, phenyl-$(C_1-C_6)$-alkyl and phenyloxy-$(C_1-C_6)$-alkyl wherein the phenylgroup may be substituted with $(C_1-C_6)$alkyl, $(C_1-C_6)$alkoxy or halogen, and naphtyl-$(C_1-C_6)$-alkyl,
$R_2$ and $R_3$ are both independently hydrogen or halogen,
$R_4$ is a biolabile ester forming group,
M is a metal ion, preferably a bivalent metal ion.
n is 1, 2 or 3;
A is $CH_2$, O or S;

in an amount of between up to 50% of the total weight of the formulation,
b) a non-ionic hydrophilic surfactant ingredient, which is in the liquid form between 15° and 30°C, in an amount of between 20% and 70 % of the total weight of the formulation and
c) a pharmaceutically acceptable organic polymer or mixture of polymers, which polymer or mixture of polymers is in a liquid form above 60°C and in a solid form below 30°C, in an amount of between 5 % and 70% of the total weight of the formulation, and
d) optionally comprises a disintegrating agent in an amount of between 1% and 10% of the total weight of the formulation.

**2.** An oral immediate release formulation with enhanced bio-availability comprising a solid homogeneous and thermodynamically stable solution of a poorly water-soluble biologically active substance, **characterised in that** said solid solution comprises:

a) a compound of the general formula

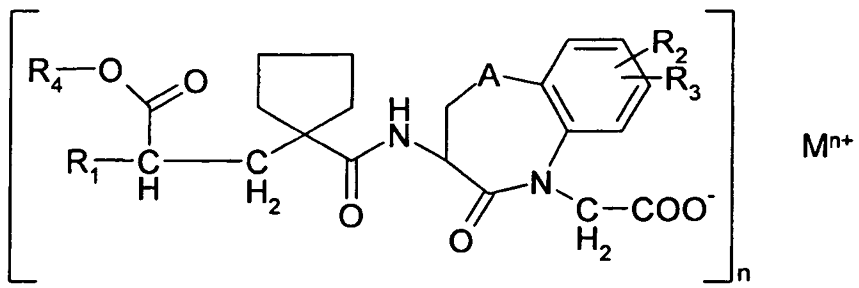

**(I)**

wherein:

$R_1$ is a selected from the group consisting of $(C_1-C_6)$alkoxy$(C_1-C_6)$alkyl which may be substituted by a $(C_1-C_6)$alkoxy, phenyl-$(C_1-C_6)$-alkyl and phenyloxy-$(C_1-C_6)$-alkyl wherein the phenylgroup may be substituted with $(C_1-C_6)$alkyl, $(C_1-C_6)$alkoxy or halogen, and naphtyl-$(C_1-C_6)$-alkyl,
$R_2$ and $R_3$ are both independently hydrogen or halogen,
$R_4$ is a biolabile ester forming group,
M is a metal ion, preferably a bivalent metal ion.
n is 1, 2 or 3;
A is $CH_2$;

in an amount of between up to 50% of the total weight of the formulation,
b) a non-ionic hydrophilic surfactant ingredient, which is in the liquid form between 15° and 30°C, in an amount of between 20% and 70 % of the total weight of the formulation and
c) a pharmaceutically acceptable organic polymer or mixture of polymers, which polymer or mixture of polymers is in a liquid form above 60°C and in a solid form below 30°C, in an amount of between 5 % and 70% of the total weight of the formulation, and
d) optionally comprises a disintegrating agent in an amount of between 1% and 10% of the total weight of the formulation.

**3.** An oral immediate release formulation according to claims 1 or 2, **characterised in that** the ratio between the active substance and the non-ionic hydrophilic surfactant ingredient is between 1 : 0.75 and 1 : 5 and the ratio between the surfactant and the pharmaceutically acceptable organic polymer or mixture of polymers is between 1 : 4 and 1 : 0.05.

**4.** An oral immediate release formulation according to claim 3, **characterized in that** the ratio between the surfactant and the pharmaceutically acceptable organic polymer or mixture of polymers is between 1 : 1.5 and 1 : 0.1.

**5.** An immediate release formulation according to claims 1 - 4, **characterized in that** the non-ionic hydrophilic surfactant ingredient is selected from the group consisting of polyoxyethylene glycol sorbitan fatty acid esters (polysorbates) and non hydrogenated polyoxyethylene castor oil derivatives, said non-ionic hydrophilic surfactant having a hydrophilic-lipophilic balance (HLB) value of between 14 and 16.

**6.** An immediate release formulation according to claim 5, **characterized in that** non-ionic hydrophilic surfactant ingredient is Tween®, preferably Tween® 80 or Chremophor EL.

**7.** An immediate release formulation according to claims 1 - 6, **characterized in that** the pharmaceutically acceptable organic polymer is a polyethylene glycol or a mixture of polyethylene glycols, each with a molecular weight of between 1000 and 50000 Daltons, preferably between 4000 and 10000 Daltons.

**8.** An immediate release formulation according to claims 1 - 6, **characterized in that** the pharmaceutically acceptable organic polymer is a polyvinyl pyrrolidon or a mixture of polyvinyl pyrrolidons with molecular weight range of 2500 up to 3000000 Daltons or a polyvinyl alcohol or a mixture of polyvinyl alcohols with molecular weight range of 30000 up to 200000 Daltons.

**9.** An immediate release formulation according to claim 1 - 8 wherein M in the general formula (I) is calcium in its 2+ form.

**10.** An immediate release formulation according to claims 1 - 9, **characterized in that** said poorly water-soluble active substance is the calcium salt of 1H-1-Benzazepine-1-acetic acid, 3-[[[1-[2-(ethoxycarbonyl)-4-phenylbutyl]cyclopentyl]carbonyl]-amino]-2,3,4,5-tetrahydro-2-oxo-, preferably in its 3S,2'R form.

**11.** Formulation according to claims 1-10 contained in a hard gelatine or soft gelatine capsule.

**12.** A method of preparing a formulation according to claims 1-11, **characterized in that** a) the non-ionic hydrophilic surfactant ingredient is mixed with the pharmaceutically acceptable organic polymer or mixture of polymers at between 50-100°C,

b) the compound of the general formula (I) is added and dissolved at said temperature,
c) the resulting mixture is optionally filled into a capsule and
d) the resulting mixture is solidified at room temperature.

**13.** A method of preparing a formulation according to claims 1-11, **characterized in that** a) the non-ionic hydrophilic surfactant ingredient is mixed with the pharmaceutically acceptable organic polymer or mixture of polymers and the compound of the general formula (I) at between 50-100°C,

b) the resulting mixture is optionally filled into a capsule and
c) the resulting mixture is solidified at room temperature.

**Patentansprüche**

**1.** Orale Sofortabgabeformulierung mit erhöhter Bioverfügbarkeit, umfassend eine feste, homogene und thermodynamisch stabile Lösung einer schlecht wasserlöslichen, biologisch wirksamen Substanz, **dadurch gekennzeichnet, dass** besagte feste Lösung umfasst:

a) eine Verbindung der allgemeinen Formel

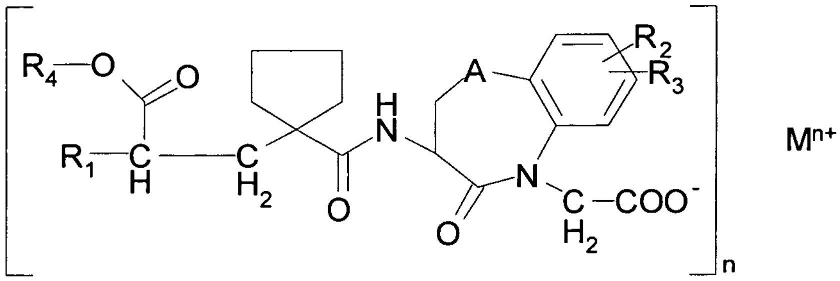

(I),

worin:

$R_1$ ausgewählt wird aus der Gruppe bestehend aus $(C_{1-}C_6)$Alkoxy$(C_1-C_6)$alkyl, welches substituiert sein kann durch ein $(C_1-C_6)$Alkoxy, Phenyl-$(C_1-C_6)$-alkyl und Phenyloxy-$(C_1-C_6)$-alkyl, worin die Phenylgruppe substituiert sein kann mit $(C_1-C_6)$Alkyl, $(C_1-C_6)$Alkoxy oder Halogen, und Naphthyl-$(C_1-C_6)$-alkyl,
$R_2$ und $R_3$ beide unabhängig Wasserstoff oder Halogen darstellen,
$R_4$ eine biologisch instabile Ester-bildende Gruppe darstellt,
M für ein Metallion steht, vorzugsweise ein zweiwertiges Metallion,
n 1, 2 oder 3 ist;
A für $CH_2$, O oder S steht;

in einer Menge von zwischen bis zu 50% des Gesamtgewichts der Formulierung,
b) einen nicht-ionischen, hydrophilen Surfactant-Inhaltsstoff, welcher sich zwischen 15° und 30°C in flüssiger Form befindet, in einer Menge von zwischen 20% und 70% des Gesamtgewichts der Formulierung und
c) ein pharmazeutisch annehmbares organisches Polymer oder Gemisch aus Polymeren, welches Polymer oder Gemisch aus Polymeren sich über 60°C in einer flüssigen Form und unter 30°C in einer festen Form befindet, in einer Menge von zwischen 5% und 70% des Gesamtgewichts der Formulierung, und
d) gegebenenfalls ein Aufschlussmittel in einer Menge von zwischen 1% und 10% des Gesamtgewichts der Formulierung umfasst.

**2.** Orale Sofortabgabeformulierung mit erhöhter Bioverfügbarkeit, umfassend eine feste, homogene und thermodynamisch stabile Lösung einer schlecht wasserlöslichen, biologisch wirksamen Substanz, **dadurch gekennzeichnet, dass** besagte feste Lösung umfasst:

a) eine Verbindung der allgemeinen Formel

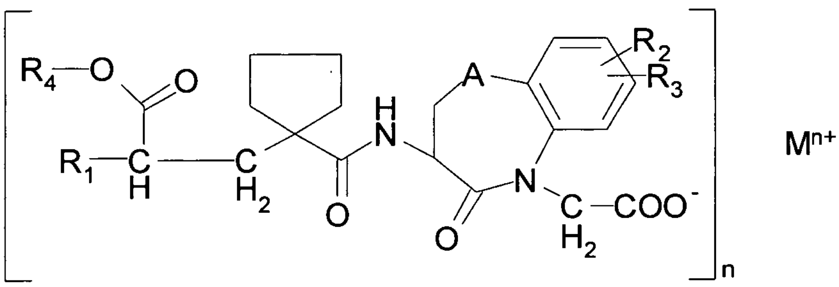

(I),

worin:

$R_1$ ausgewählt wird aus der Gruppe bestehend aus $(C_{1-}C_6)$Alkoxy$(C_1-C_6)$alkyl, welches substituiert sein kann durch ein $(C_1-C_6)$Alkoxy, Phenyl-$(C_1-C_6)$-alkyl und Phenyloxy-$(C_1-C_6)$-alkyl, worin die Phenylgruppe

substituiert sein kann mit ($C_1$-$C_6$)Alkyl, ($C_1$-$C_6$)Alkoxy oder Halogen, und Naphthyl-($C_1$-$C_6$)-alkyl,
$R_2$ und $R_3$ beide unabhängig Wasserstoff oder Halogen darstellen,
$R_4$ eine biologisch instabile Ester-bildende Gruppe darstellt,
M für ein Metallion steht, vorzugsweise ein zweiwertiges Metallion,
n 1, 2 oder 3 ist;
A für $CH_2$ steht;

in einer Menge von zwischen bis zu 50% des Gesamtgewichts der Formulierung,
b) einen nicht-ionischen, hydrophilen Surfactant-Inhaltsstoff, welcher sich zwischen 15° und 30°C in flüssiger Form befindet, in einer Menge von zwischen 20% und 70% des Gesamtgewichts der Formulierung und
c) ein pharmazeutisch annehmbares organisches Polymer oder Gemisch aus Polymeren, welches Polymer oder Gemisch aus Polymeren sich über 60°C in einer flüssigen Form und unter 30°C in einer festen Form befindet, in einer Menge von zwischen 5% und 70% des Gesamtgewichts der Formulierung, und
d) gegebenenfalls ein Aufschlussmittel in einer Menge von zwischen 1% und 10% des Gesamtgewichts der Formulierung umfasst.

**3.** Orale Sofortabgabeformulierung gemäß Anspruch 1 oder 2, **dadurch gekennzeichnet, dass** das Verhältnis zwischen der wirksamen Substanz und dem nicht-ionischen, hydrophilen Surfactant-Inhaltsstoff zwischen 1:0,75 und 1:5 ist, und das Verhältnis zwischen dem Surfactant und dem pharmazeutisch annehmbaren organischen Polymer oder Gemisch aus Polymeren zwischen 1:4 und 1:0,05 ist.

**4.** Orale Sofortabgabeformulierung gemäß Anspruch 3, **dadurch gekennzeichnet, dass** das Verhältnis zwischen dem Surfactant und dem pharmazeutisch annehmbaren organischen Polymer oder Gemisch aus Polymeren zwischen 1:1,5 und 1:0,1 ist.

**5.** Sofortabgabeformulierung gemäß Ansprüchen 1-4, **dadurch gekennzeichnet, dass** der nicht-ionische, hydrophile Surfactant-Inhaltsstoff ausgewählt wird aus der Gruppe bestehend aus Polyoxy-ethylenglycol-sorbitanfettsäureester (Polysorbaten) und nicht-hydrierten Polyoxyethylen-Castoröl-Derivaten, wobei besagter nicht-ionischer, hydrophiler Surfactant einen Hydrophil-Lipophilen-Gleichgewichts- (HLB)-Wert von zwischen 14 und 16 hat.

**6.** Sofortabgabeformulierung gemäß Anspruch 5, **dadurch gekennzeichnet, dass** der nicht-ionische, hydrophile Surfactant-Inhaltsstoff Tween®, vorzugsweise Tween® 80 oder Chremophor EL ist.

**7.** Sofortabgabeformulierung gemäß Ansprüchen 1-6, **dadurch gekennzeichnet, dass** das pharmazeutisch annehmbare organische Polymer ein Polyethylenglycol oder ein Gemisch aus Polyethylenglycolen ist, jedes mit einem Molekulargewicht von zwischen 1000 und 50000 Dalton, vorzugsweise zwischen 4000 und 10000 Dalton.

**8.** Sofortabgabeformulierung gemäß Ansprüchen 1-6, **dadurch gekennzeichnet, dass** das pharmazeutisch annehmbare organische Polymer ein Polyvinylpyrrolidon oder ein Gemisch aus Polyvinylpyrrolidonen mit einem Molekulargewichtsbereich von 2500 bis zu 3000000 Dalton oder ein Polyvinylalkohol oder ein Gemisch aus Polyvinylalkoholen mit einem Molekulargewichtsbereich von 30000 bis zu 200000 Dalton ist.

**9.** Sofortabgabeformulierung gemäß Anspruch 1-8, worin M in der allgemeinen Formel (I) für Calcium in seiner 2+ Form steht.

**10.** Sofortabgabeformulierung gemäß Ansprüchen 1-9, **dadurch gekennzeichnet, dass** besagte schlecht wasserlösliche, wirksame Substanz das Calciumsalz von 1H-1-Benzazepin-1-essigsäure, 3-[[[1-[2-(Ethoxycarbonyl)-4-phenylbutyl]cyclopentyl]carbonyl]-amino]-2,3,4,5-tetrahydro-2-oxo, vorzugsweise in seiner 3S,2'R-Form ist.

**11.** Formulierung gemäß Ansprüchen 1-10, enthalten in einer Hartgelatine- oder Weichgelatine-Kapsel.

**12.** Verfahren zum Herstellen einer Formulierung gemäß Ansprüchen 1-11, **dadurch gekennzeichnet, dass** a) der nicht-ionische, hydrophile Surfactant-Inhaltsstoff mit dem pharmazeutisch annehmbaren organischen Polymer oder Gemisch aus Polymeren bei zwischen 50-100°C gemischt wird,

b) die Verbindung der allgemeinen Formel (I) zugegeben und bei besagter Temperatur gelöst wird,
c) das sich ergebende Gemisch gegebenenfalls in eine Kapsel gefüllt wird und
d) das sich ergebende Gemisch bei Raumtemperatur verfestigt wird.

**13.** Verfahren zum Herstellen einer Formulierung gemäß Ansprüchen 1-11, **dadurch gekennzeichnet, dass** a) der nicht-ionische, hydrophile Surfactant-Inhaltsstoff mit dem pharmazeutisch annehmbaren organischen Polymer oder Gemisch aus Polymeren und der Verbindung der allgemeinen Formel (I) bei zwischen 50-100°C gemischt wird,

b) das sich ergebende Gemisch gegebenenfalls in eine Kapsel gefüllt wird und
c) das sich ergebende Gemisch bei Raumtemperatur verfestigt wird.

**Revendications**

**1.** Formulation orale à libération immédiate ayant une biodisponibilité accrue comprenant une solution solide homogène et thermodynamiquement stable d'une substance biologiquement active médiocrement soluble dans l'eau, **caractérisée en ce que** ladite solution solide comprenant :

a) un composé de formule générale

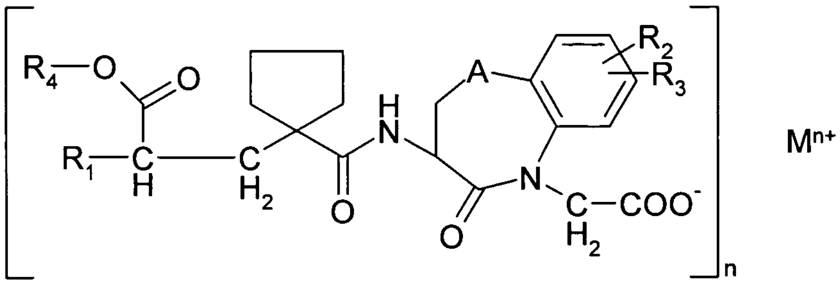

(I)

dans laquelle :

$R_1$ est choisi dans le groupe constitué par un groupe alcoxy (en $C_1$ à $C_6$)-alkyle (en $C_1$ à $C_6$) qui peut être substitué par un groupe alcoxy (en $C_1$ à $C_6$), phényl-alkyle (en $C_1$ à $C_6$) et phényloxy-alkyle (en $C_1$ à $C_6$) dans lequel le groupe phényle peut être substitué par alkyle (en $C_1$ à $C_6$), alcoxy (en $C_1$ à $C_6$) ou halogène, et un groupe naphtyl-alkyle (en $C_1$ à $C_6$),
$R_2$ et $R_3$ sont tous les deux indépendamment un atome d'hydrogène ou d'halogène,
$R_4$ est un groupe formant un ester biolabile,
M est un ion métallique, de préférence un ion métallique divalent,
n vaut 1, 2 ou 3,
A est $CH_2$, O ou S,

en une quantité allant jusqu'à 50% de poids total de la formulation ;
b) un ingrédient tensioactif hydrophile non ionique, qui est sous forme liquide entre 15° et 30° C, en une quantité comprise entre 20 % et 70 % du poids total de formulation, et
c) un polymère organique ou mélange de polymères pharmaceutiquement acceptable, lequel polymère ou mélange de polymères est sous forme liquide au-dessus de 60° C et sous forme solide en dessous de 30° C, en une quantité comprise entre 5 % et 70 % du poids total de la formulation, et
d) comprend facultativement un agent désintégrant une quantité comprise entre 1 % et 10 % du poids total de la formulation.

**2.** Formulation orale à libération immédiate ayant une biodisponibilité accrue comprenant une solution solide homogène et thermodynamiquement stable d'une substance biologiquement active médiocrement soluble dans l'eau, **caractérisée en ce que** ladite solution solide comprenant :

a) un composé de formule générale

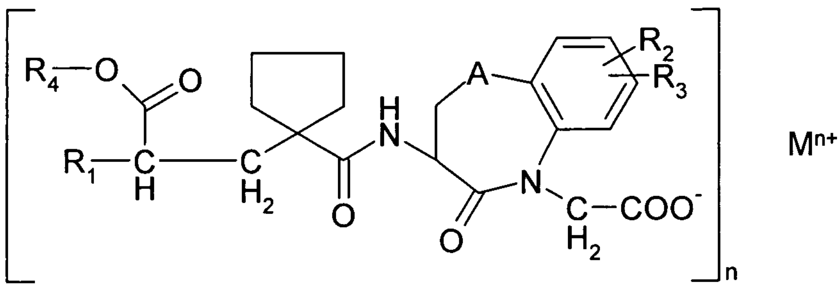

(I)

dans laquelle :

$R_1$ est choisi dans le groupe constitué par un groupe alcoxy (en $C_1$ à $C_6$)-alkyle (en $C_1$ à $C_6$) qui peut être substitué par un groupe alcoxy (en $C_1$ à $C_6$), phényl-alkyle (en $C_1$ à $C_6$) et phényloxy-alkyle (en $C_1$ à $C_6$) dans lequel le groupe phényle peut être substitué par alkyle (en $C_1$ à $C_6$), alcoxy (en $C_1$ à $C_6$) ou halogène, et un groupe naphtyl-alkyle (en $C_1$ à $C_6$),
$R_2$ et $R_3$ sont tous les deux indépendamment un atome d'hydrogène ou d'halogène,
$R_4$ est un groupe formant un ester biolabile,
M est un ion métallique, de préférence un ion métallique divalent,
n vaut 1, 2 ou 3,
A est $CH_2$,

en une quantité allant jusqu'à 50% de poids total de la formulation ;
b) un ingrédient tensioactif hydrophile non ionique, qui est sous forme liquide entre 15° et 30° C, en une quantité comprise entre 20 % et 70 % du poids total de formulation, et
c) un polymère organique ou mélange de polymères pharmaceutiquement acceptable, lequel polymère ou mélange de polymères est sous forme liquide au-dessus de 60° C et sous forme solide en dessous de 30° C, en une quantité comprise entre 5 % et 70 % du poids total de la formulation, et
d) comprend facultativement un agent désintégrant une quantité comprise entre 1 % et 10 % du poids total de la formulation.

**3.** Formulation orale à libération immédiate selon la revendication 1 ou 2, **caractérisée en ce que** le rapport entre la substance active et l'ingrédient tensioactif hydrophile non ionique est compris entre 1 : 0,75 et 1 : 5 et le rapport entre l'agent tensioactif et le polymère organique ou mélange de polymères pharmaceutiquement acceptable est compris entre 1 : 4 et 1 : 0,05.

**4.** Formulation orale à libération immédiate selon la revendication 3, **caractérisée en ce que** le rapport entre l'agent tensioactif et le polymère organique ou mélange de polymères pharmaceutiquement acceptable est compris entre 1 : 1,5 et 1 : 0,1.

**5.** Formulation à libération immédiate selon les revendications 1 à 4, **caractérisée en ce que** l'ingrédient tensioactif hydrophile non ionique est choisi dans le groupe constitué par les esters d'acide gras sorbitan de polyoxyéthylène glycol (polysorbates) et les dérivés de polyoxyéthylène huile de ricin non hydrogénés, ledit agent tensioactif hydrophile non ionique ayant une valeur d'équilibre hydrophile-lipophile (HLB) comprise entre 14 et 16.

**6.** Formulation à libération immédiate selon la revendication 5, **caractérisée en ce que** l'agent tensioactif hydrophile non ionique est un Tween®, de préférence de Tween 80® ou le Chremophor EL.

**7.** Formulation à libération immédiate selon les revendications 1 à 7, **caractérisée en ce que** le polymère organique pharmaceutiquement acceptable est un polyéthylène glycol ou un mélange de polyéthylène glycols, chacun avec une masse moléculaire comprise entre 1 000 et 50 000 Daltons, de préférence entre 4 000 et 10 000 Daltons.

**8.** Formulation à libération immédiate selon les revendications 1 à 6, **caractérisée en ce que** le polymère organique

pharmaceutiquement acceptable est une polyvinylpyrrolidone ou un mélange de polyvinylpyrrolidones avec une gamme de masse moléculaire de 2 500 jusqu'à 3 000 000 Daltons ou un polyalcool vinylique ou un mélange de polyalcool vinyliques avec une gamme de masse moléculaire de 30 000 jusqu'à 200 000 Daltons.

**9.** Formulation à libération immédiate selon les revendications 1 à 8, dans laquelle M est le calcium sous sa forme 2+.

**10.** Formulation à libération immédiate selon les revendications 1 à 9, **caractérisée en ce que** ladite substance active médiocrement soluble dans l'eau est le sel de calcium de l'acide 1H-1-benzazépine-1-acétique, 3-[[[1-(2-éthoxycarbonyl)-4-phénylbutyl]cyclopentyl]carbonyl]amino]-2,3,4,5-tétrahydro-2-oxo-, de préférence sous sa forme 3S, 2'R.

**11.** Formulation selon les revendications 1 à 10 contenue dans une capsule gélatineuse dure ou gélatineuse molle.

**12.** Procédé de préparation d'une formulation selon les revendications 1 à 11, **caractérisé en ce que** a) l'ingrédient tensioactif hydrophile non ionique est mélangé avec le polymère organique ou mélange de polymères pharmaceutiquement acceptable entre 50 et 100° C,

b) le composé de formule générale (I) est ajouté et dissout à ladite température,
c) le mélange résultant est facultativement rempli dans une capsule et
d) le mélange résultant est solidifié à température ambiante.

**13.** Procédé de préparation d'une formulation selon les revendications 1 à 11, **caractérisé en ce que** a) l'ingrédient tensioactif hydrophile non ionique est mélangé avec le polymère organique ou mélange de polymères pharmaceutiquement acceptable et le composé de formule générale (I) entre 50 et 100° C,

b) le mélange résultant est facultativement rempli dans une capsule et
c) le mélange résultant est solidifié à température ambiante.

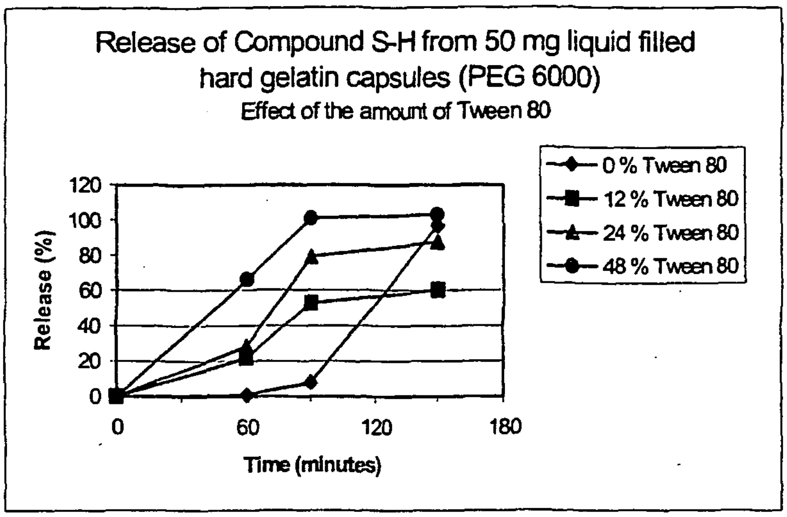


**Figure 1.** Dissolution of the compositions A,B, C and D from Table 1.

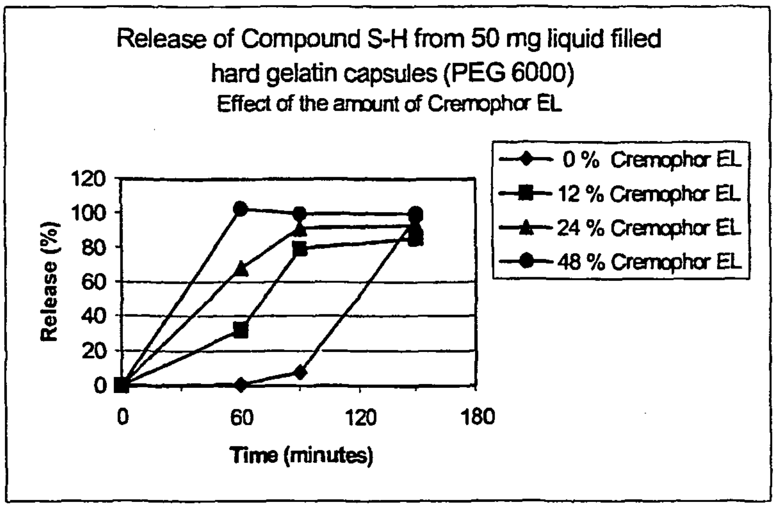


**Figure 2.** Dissolution of the compositions A, E, F and G from Table 1

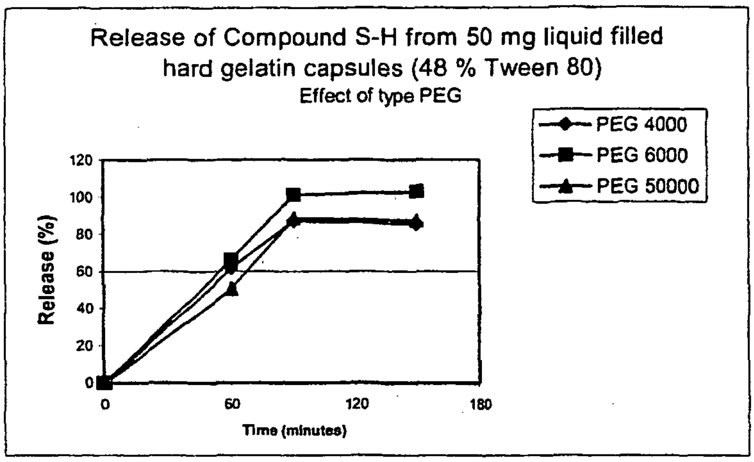


**Figure 3** Influence of type of polyethylene glycol on the release of the active substance

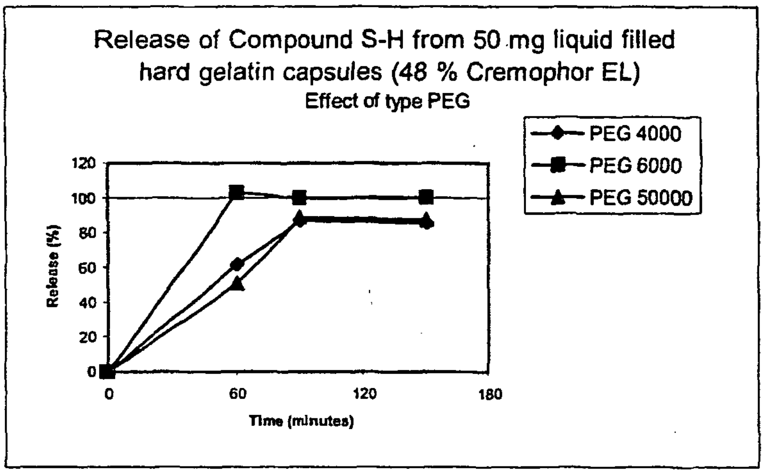


**Figure 4** Influence of type of polyethylene glycol on the release of the active substance

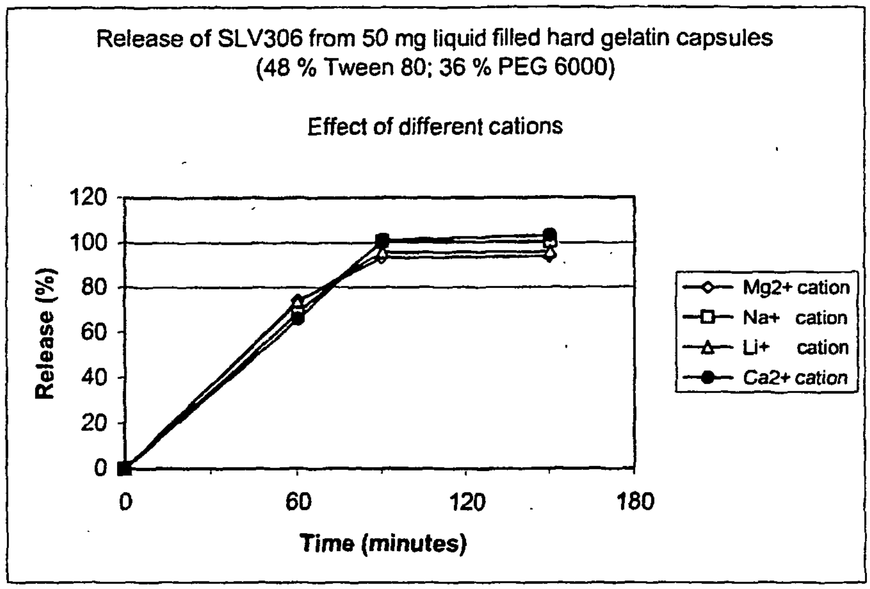


**Figure 5** Effect of different cations on release of the active substance.

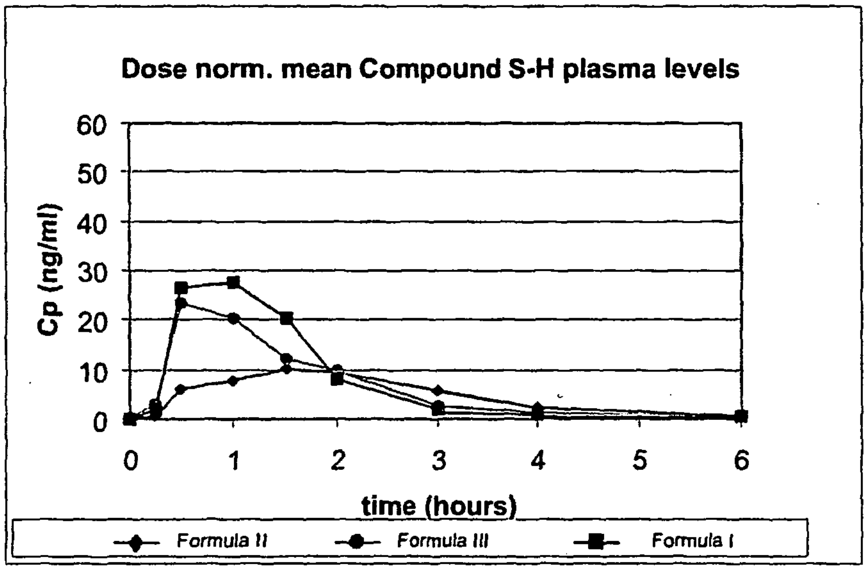


**Figure 6** Mean plasma levels of the cross-over study with Compound S.